(19) Europäisches Patentamt
European Patent Office
Office européen des brevets

(11) EP 2 529 769 B1

# (12) EUROPEAN PATENT SPECIFICATION

(45) Date of publication and mention of the grant of the patent:
**18.01.2017 Bulletin 2017/03**

(21) Application number: **11734803.7**

(22) Date of filing: **24.01.2011**

(51) Int Cl.:
***A61M 1/18*** *(2006.01)*

(86) International application number:
**PCT/JP2011/051226**

(87) International publication number:
**WO 2011/090197 (28.07.2011 Gazette 2011/30)**

## (54) HOLLOW FIBER MEMBRANE-BASED BLOOD PURIFICATION APPARATUS

BLUTREINIGUNGSVORRICHTUNG MIT HOHLFASERMEMBRANEN

DISPOSITIF DE PURIFICATION DE SANG À MEMBRANE À FIBRES CREUSES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2010 JP 2010013066**

(43) Date of publication of application:
**05.12.2012 Bulletin 2012/49**

(73) Proprietor: **Asahi Kasei Medical Co., Ltd.**
**Chiyoda-ku**
**Tokyo 101-8101 (JP)**

(72) Inventors:
- **NISHIZAKI, Hiroshi**
  **Tokyo 101-8101 (JP)**
- **SATOH, Junya**
  **Tokyo 101-8101 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**EP-A2- 0 103 816**
**EP-A2- 0 923 955**
**WO-A2-2007/018242**
**JP-A- 7 178 166**
**JP-A- 2006 296 931**
**JP-A- 2008 093 228**
**JP-A- 2009 022 635**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 529 769 B1

**Description**

Technical Field

The present invention relates to a hollow fiber membrane-based blood purification apparatus.

Background Art

Heretofore, hollow fiber membrane-based blood processing apparatuses using selectively permeable membranes have been used widely in the technical field of extracorporeal blood circulation, for example, hemodialysis, oxygen supply to blood during open-heart surgery, or plasma separation.

Blood processing membranes made of polysulfone-based resins (hereinafter, abbreviated to polysulfones) as materials are widely used, particularly, in the field of blood processing membranes such as dialysis membranes, gas exchange membranes, and blood component separation membranes.

Since these polysulfones alone, however, are highly hydrophobic and insufficiently offer blood compatibility, their complexes with hydrophilic polymers such as polyvinylpyrrolidone are generally used as materials constituting blood processing membranes.

In recent years, attempts have also been made to impart the function of alleviating oxidative stress observed in patients receiving long-term dialysis, to the blood processing membranes already having the function as separation membranes. Examples of such attempts may include to eliminate peroxide causative of oxidative stress by use of separation membranes and to allow organisms to recover antioxidative effect. Specifically, there has been proposed a hollow fiber membrane-based blood purification apparatus in which the surface of a dialysis membrane is covered with vitamin E having various physiological effects such as *in vivo* antioxidative effect, biomembrane stabilizing effect, and platelet aggregation inhibitory effect (see e.g., Patent Document 1).

Meanwhile, hollow fiber membrane-based blood purification apparatuses are usually sterilized under hermetically sealed package conditions in actual use in consideration of their use application.

For example, gaseous sterilization using ethylene oxide gas or the like, autoclaving, and radiation sterilization using $\gamma$ rays, electron beam, or the like are known as methods for sterilizing the hollow fiber membrane-based blood purification apparatus.

Unfortunately, the gaseous sterilization method using ethylene oxide gas is harmful to human bodies due to undesirable residues of ethylene oxide gas.

Alternatively, the autoclaving method may significantly reduce the performance of the hollow fiber membrane-based blood purification apparatus during sterilization, depending on materials constituting it. In addition, autoclaving, when applied to a vitamin E-immobilized polysulfone membrane, may cause the local aggregation of vitamin E and generate cracks in the polysulfone membrane, resulting in the leakage of blood (see e.g., Patent Document 2).

On the other hand, the radiation sterilization method is free from these problems, such as residues of ethylene oxide gas or the leakage of blood resulting from generated cracks, and is thus preferable for sterilization.

These hollow fiber membrane-based blood purification apparatuses are broadly classified into two types: wet type in which the inside of the hollow fiber membrane or the gap between the hollow fiber membrane and a container is filled with an aqueous medium; and a dry type in which such space is not filled with an aqueous medium.

It is known that, among the hollow fiber membrane-based blood purification apparatuses of this dry type, those of type having a hollow fiber membrane with a low water content, i.e., a small percent or lower of water content (dry type in the narrow sense) exhibits reduction in blood compatibility, when radiation-sterilized, because a hydrophilic polymer constituting the hollow fiber membrane is deteriorated or eluted.

For preventing such reduction in blood compatibility, there has been proposed a method involving protecting a hollow fiber membrane with a wet protective agent while radiation-sterilizing the hollow fiber membrane with its neighboring oxygen concentration controlled (see e.g., Patent Document 3). This literature has proposed water, a polyhydric alcohol, or the like as the wet protective agent. Such hollow fiber membrane-based blood purification apparatuses having a hollow fiber membrane wetted to some extent with a wet protective agent are also called semidry or half-wet type differentiated from dry type in the narrow sense. The hollow fiber membrane-based blood purification apparatuses of semidry or half-wet type and of dry type in the narrow sense have excellent properties in common in terms of distribution including transportation and storage in such a way that the resulting products have a smaller weight than that of wet type and those containing a wetting agent are hardly frozen under low temperature conditions. Thus, both of them are collectively called dry type in the present specification.

As described above, the hollow fiber membrane-based blood purification apparatus of dry type has excellent properties in terms of distribution including transportation and storage and however requires protecting a hollow fiber membrane with a wet protective agent while radiation-sterilizing it with the oxygen concentration controlled, for improving blood compatibility. In this regard, the apparatus is disadvantageously low productive.

Prior Art Documents

Patent Document

Patent Document 1: Japanese Patent Laid-Open No. 7-178166
Patent Document 2: Japanese Patent Laid-Open No. 2006-296931
Patent Document 3: Japanese Patent Laid-Open No. 2008-93228

EP 0923955 discloses a hollow fiber membrane based blood purification apparatus comprising a hollow fiber membrane comprising a polysulfone-based resin, a hydrophilic polymer, a fat-soluble vitamin and wherein the hollow fiber membrane is covered with an aqueous Hydrophilic compound solution.

Summary of Invention

Problems to be Solved by the Invention

Thus, an object of the present invention is to provide a hollow fiber membrane-based blood purification apparatus that has a high level of blood compatibility, exhibits excellent antioxidative properties, eliminates the need for oxygen concentration control during radiation sterilization, and is highly productive.

Means for Solving the Problems

The present inventors have conducted diligent studies to attain the object and consequently found that an effective approach for this purpose is to protect, with a particular amount of an aqueous hydrophilic compound solution or water, a hollow fiber membrane having a particular amount of a fat-soluble vitamin immobilized in the surface. The present inventors have thereby successfully obtained a hollow fiber membrane-based blood purification apparatus that has a high level of blood compatibility, exhibits excellent antioxidative properties, eliminates the need for oxygen concentration control during radiation sterilization, and is highly productive. In this way, the present invention has been completed.

Specifically, the present invention is as follows:

[1] A hollow fiber membrane-based blood purification apparatus comprising a hollow fiber membrane containing a polysulfone-based resin, a hydrophilic polymer, and a fat-soluble vitamin, the hollow fiber membrane-based blood purification apparatus being radiation-sterilized, wherein

the amount of the fat-soluble vitamin on the surface of the hollow fiber membrane is 1.5 mg or more and 23 mg or less, based on 1 g of the hollow fiber membrane, wherein the amount of the fat-soluble vitamin is determined as described in the description, and
the surface of the hollow fiber membrane is covered with a mixture of water and a polyhydric alcohol at a content of 60 % or more, based on the dry weight of the hollow fiber membrane, to the saturated liquid content or less, characterized in that the weight ratio of the polyhydric alcohol to water in the mixture is 1.3 times or more and 4.8 times or less, and

wherein the surface of the hollow fiber membrane includes the inner and outer surfaces of the hollow fiber membrane.

[2] The hollow fiber membrane-based blood purification apparatus according to [1], wherein the amount of the fat-soluble vitamin on the surface of the hollow fiber membrane is 1.5 mg or more and 18 mg or less, based on 1 g of the hollow fiber membrane.

[3] The hollow fiber membrane-based blood purification apparatus according to [1], wherein the attachment rate of water constituting the mixture of water and polyhydric alcohol to the hollow fiber membrane is 40 % or more and less than 100 %, based on the dry weight of the hollow fiber membrane, and the attachment rate of the polyhydric alcohol to the hollow fiber membrane is 20 % or more and 300 % or less, based on the dry weight of the hollow fiber membrane, wherein the attachment rates of water and the polyhydric alcohol are determined as described in the description.

Advantageous Effects of Invention

According to the present invention, a hollow fiber membrane-based blood purification apparatus that has a high level of blood compatibility and exhibits excellent antioxidative properties is obtained.
The obtained hollow fiber membrane-based blood purification apparatus further eliminates the need for lowering oxygen concentrations during radiation sterilization to achieve a simplified production process and is also excellent in rational production.

Brief Description of Drawings

[Figure 1] Figure 1 shows a model diagram of a hollow fiber membrane before covering with an aqueous hydrophilic compound solution.
[Figure 2] Figure 2 shows a model diagram of a hollow fiber membrane after covering with an aqueous hydrophilic compound solution according to conventional techniques.
[Figure 3] Figure 3 shows a model diagram of a hollow fiber membrane after covering with an aqueous hydrophilic compound solution according to Examples of the present invention.
[Figure 4] Figure 4 shows a diagram showing the relationship between the amount of a fat-soluble vitamin in hollow fiber membrane surface and LDH.

Mode for Carrying Out the Invention

Hereinafter, an embodiment of the present invention (hereinafter, referred to as the "present embodiment") will be described with reference to the drawings.
In the drawings, the same reference numerals will be used to designate the same components, so that overlapping descriptions will be omitted. Positional relationships indicated by the terms "upper", "lower", "right", "left", and the like are based on those shown in the drawings, unless otherwise specified. In addition, the dimensional ratios of the drawings are not intended to be limited to those shown in the drawings.

[Hollow fiber membrane-based blood purification apparatus]

A hollow fiber membrane-based blood purification apparatus comprising a hollow fiber membrane containing a polysulfone-based resin, a hydr-ophilic polymer, and a fat-soluble vitamin, the hollow fiber membrane-based blood purification apparatus being radiation-sterilized, wherein

the amount of the fat-soluble vitamin on the surface of the hollow fiber membrane is 1.5 mg or more and 23 mg or less, based on 1 g of the hollow fiber membrane, wherein the amount of the fat-soluble vitamin is determined as described in the description, and
the surface of the hollow fiber membrane is covered with a mixture of water and a polyhydric alcohol at a content of 60 % or more, based on the dry weight of the hollow fiber membrane to the saturated liquid content or less, characterized in that the weight ratio of the polyhydric alcohol to water in the mixture is 1.3 times or more and 4.8 times or less, and

wherein the surface of the hollow fiber membrane includes the inner and outer surfaces of the hollow fiber membrane.

(Hollow fiber membrane)

The hollow fiber membrane constituting the hollow fiber membrane-based blood purification apparatus of the present embodiment contains a polysulfone-based resin, a hydrophilic polymer, and a fat-soluble vitamin.

<Polysulfone-based resin>

The polysulfone-based resin (hereinafter, also referred to as PSf) is a generic name for polymer compounds having a sulfone bond. Examples thereof may include polysulfone-based polymers having repeat units represented by the formulas (1) to (5) shown below.
In the formulas (1) to (5) below, n is preferably 10 or larger.
The bisphenol-based polysulfone polymer of the formula (1) below is commercially available under the trade

name of "Udel" from Solvay Advanced Polymers or under the trade name of "Ultrason" from BASF Corp. and is found to be of several types, any of which may be used without particular limitations, depending on the degree of polymerization.

[Formula 1]

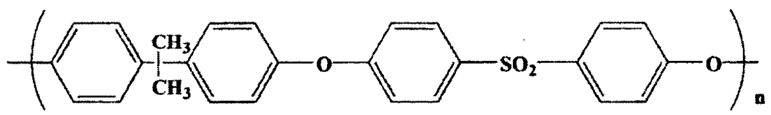

(1)

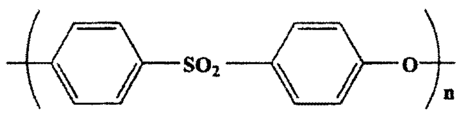

(2)

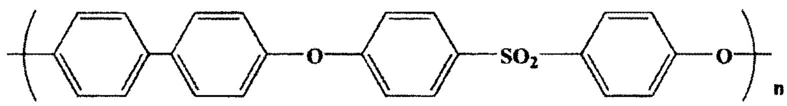

(3)

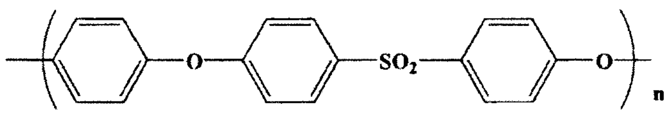

(4)

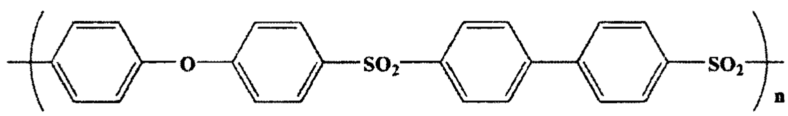

(5)

<Hydrophilic polymer>

Examples of the hydrophilic polymer may include polyvinylpyrrolidone (hereinafter, also referred to as PVP), polyethylene glycol, polyvinyl alcohol, and polypropylene glycol. PVP is preferably used from the viewpoint of spinning stability and affinity for PSf described above.

PVP is also found to be of several types depending on the degree of polymerization. For example, K-15, K-30, and K-90 differing in molecular weight are available under the trademark of "Plasdone" from ISP Ltd., and any of them may be used.

<Fat-soluble vitamin>

The fat-soluble vitamin generally refers to a vitamin that is hardly dissolved in water and is dissolved in alcohols or fats and oils.

Examples thereof may include vitamin A, vitamin D, vitamin E, vitamin K, and ubiquinone. Vitamin E is particularly preferable.

Examples of the vitamin E may include α-tocopherol, α-tocopherol acetate, α-tocopherol nicotinate, β-tocopherol, γ-tocopherol, and δ-tocopherol. α-Tocopherol is particularly preferable because of having various physiological effects such as *in vivo* antioxidative effect, biomembrane stabilizing effect, and platelet aggregation inhibitory effect.

The fat-soluble vitamin has the function of alleviating oxidative stress observed in patients receiving long-term

dialysis, specifically, the function of eliminating peroxide causative of oxidative stress or allowing organisms to recover antioxidative effect. In the hollow fiber membrane-based blood purification apparatus of the present embodiment, the fat-soluble vitamin present on the surface of the hollow fiber membrane, which comes in contact with blood, produces these effects.

[Method for producing hollow fiber membrane-based blood purification apparatus]

(Method for producing hollow fiber membrane)

The hollow fiber membrane constituting the hollow fiber membrane-based blood purification apparatus of the present embodiment can be produced using a dry/wet technique of membrane formation known in the art.

First, a polysulfone-based polymer and a hydrophilic polymer are dissolved in a common solvent to prepare a dope (spinning solution).

When the hydrophilic polymer is PVP, examples of the common solvent may include solvents such as dimethylacetamide (hereinafter, referred to as DMAC), dimethyl sulfoxide, N-methyl-2-pyrrolidone, dimethylformamide, sulfolane, and dioxane. These solvents may be used alone or as a mixture of two or more thereof.

The dope may be supplemented with an additive such as water to control the pore size of the hollow fiber membrane of interest.

A hollow fiber membrane is formed using a tube-in-orifice-type spinneret. The dope and a coagulating solution for the dope are simultaneously discharged into air from the orifice of the spinneret and from a tube, respectively.

Water or a coagulating solution composed mainly of water can be used as the coagulation solution. Its composition or the like is determined according to the permeability of the hollow fiber membrane of interest. In general, a mixed solution of water with the solvent used in the dope is preferable. For example, an aqueous solution containing 0 to 65% by mass of DMAC is used.

The dope discharged from the spinneret together with the coagulating solution is traveled in an air gap portion and introduced and dipped in a coagulation bath (composed mainly of water) placed below the spinneret to complete coagulation. After a washing step, etc., the hollow fiber membrane in a wet state is wound up using a winding machine to obtain a hollow fiber membrane bundle, which is then dried. Alternatively, after the washing step, the hollow fiber membrane may be dried in a dryer to obtain a hollow fiber membrane bundle.

(Step of producing hollow fiber membrane-based blood purification apparatus)

The hollow fiber membrane-based blood purification apparatus of the present embodiment can be produced as follows: the hollow fiber membrane bundle is inserted to a tubular container having an inlet and an outlet for blood to be processed in the finally obtained hollow fiber membrane-based blood purification apparatus; a potting agent such as polyurethane is injected into both ends of the bundle to form potting layers; after sealing of both ends and curing, the redundant potting agent is removed by cutting so that the hollow portion is open at both ends; and the predetermined header is attached thereto.

(Step of adding fat-soluble vitamin to surface of hollow fiber membrane)

In the hollow fiber membrane-based blood purification apparatus of the present embodiment, the hollow fiber membrane contains a fat-soluble vitamin, wherein the amount of the fat-soluble vitamin in the surface of the hollow fiber membrane is 1.5 mg or more and 23 mg or less based on 1 g of the hollow fiber membrane.

Examples of methods for adding the fat-soluble vitamin to the hollow fiber membrane may include various methods such as: a method involving adding a fat-soluble vitamin to a membrane formation stock solution during membrane formation to thereby allow the fat-soluble vitamin to be contained in the whole hollow fiber membrane and a method involving adding a fat-soluble vitamin and a surfactant to a coagulating solution and allowing the fat-soluble vitamin to be contained on the inner surface of the hollow fiber membrane by use of the resulting coagulating solution (see e.g., Japanese Patent No. 4038583); and a method involving injecting, after module assembly, a fat-soluble vitamin solution composed of a fat-soluble vitamin and a solvent for the fat-soluble vitamin to the hollow portion of the hollow fiber membrane to thereby attach the fat-soluble vitamin to the inner surface of the hollow fiber membrane (see e.g., Japanese Patent Laid-Open No. 2006-296931). Any of these methods may be used, and the addition method is not intended to be limited to these methods or other methods.

(Step of covering hollow fiber membrane with aqueous hydrophilic compound solution)

The hollow fiber membrane constituting the hollow fiber membrane-based blood purification apparatus of the

present embodiment is covered with an aqueous hydrophilic compound solution having a 60 % with respect to its dry weight to the saturated liquid content (inclusive).

The aqueous hydrophilic compound solution refers to an aqueous solution of a hydrophilic substance.

The aqueous hydrophilic compound solution can be supplied and ejected through the predetermined inlet and outlet constituting the hollow fiber membrane-based blood purification apparatus of the present embodiment to thereby cover the hollow fiber membrane with the aqueous hydrophilic compound solution.

The covering of the inner surface of the hollow fiber membrane with the aqueous hydrophilic compound solution suffices from the viewpoint of improving blood compatibility. Additionally, the outer surface of the hollow fiber membrane or the surfaces of pores in the porous portion in the thickness portion of the hollow fiber membrane may be covered therewith in the same way as above.

The inside of pores in the hollow fiber membrane may be filled with the aqueous hydrophilic compound solution. It is however preferred that the inside of the hollow (hollow portions of the hollow fibers) should not be filled therewith because this filling causes increase in weight and liquid leakage.

(Radiation sterilization step)

The hollow fiber membrane-based blood purification apparatus of the present embodiment is radiation-sterilized.

This is because: as described above, the method using ethylene oxide gas may be harmful to human bodies due to residues of ethylene oxide gas; the autoclaving method involving high temperature and high humidity conditions, when applied to a fat-soluble vitamin-immobilized polysulfone membrane, may cause the leakage of blood attributed to the local aggregation of the fat-soluble vitamin; and by contrast, the radiation sterilization method is free from these problems.

In the radiation sterilization method, electron beam, gamma rays, x rays, or the like can be used, and any of them may be used in the present embodiment.

The exposure dose of radiation is generally 5 to 50 kGy for electron beam. It is preferred that the hollow fiber membrane-based blood purification apparatus should be exposed to radiation at a dose range of 20 to 40 kGy.

This radiation sterilization can form partial crosslinking in the hydrophilic polymer constituting the hollow fiber membrane and thereby prevent the elution of the hydrophilic polymer with favorable blood compatibility maintained.

[Properties of hollow fiber membrane-based blood purification apparatus]

(Blood compatibility)

In recent years, improvement in blood compatibility has been pursued for blood purification apparatuses. Under the present circumstances, the blood compatibility is still susceptible to improvement owing to, for example, inevitable combined use with an anticoagulant or various intercurrent complications resulting from long-term repeated use.

The present inventors developed a testing method by which a conventional blood compatibility test was conducted under more strict conditions involving long-term incubation to give an index for further improvement in blood compatibility. As in conventional techniques, a fat-soluble vitamin-free polysulfone-containing hollow fiber membrane containing a hydrophilic polymer was covered with an aqueous glycerin solution exemplified as a "wet protective agent" and further sterilized with electron beam. The obtained membrane was evaluated for its blood compatibility by the newly developed blood compatibility testing method. As a result, the blood compatibility was confirmed to be still susceptible to improvement.

In the descriptions below, a level "having room for improvement" at which blood compatibility can be found to be susceptible to improvement under the strict testing method newly developed by the present inventors is also expressed as "insufficient". However, this expression is used to intend a higher level of blood compatibility.

As a result of conducting further studies, the present inventors found that, surprisingly, a hollow fiber membrane containing a fat-soluble vitamin exhibited favorable blood compatibility by covering with an aqueous hydrophilic compound solution and sterilization with electron beam.

Further surprisingly, a hollow fiber membrane containing a fat-soluble vitamin was shown to have favorable blood compatibility even by covering with water alone and exposure to radiation.

These effects obtained in the hollow fiber membrane-based blood purification apparatus of the present embodiment cannot be imagined from previous knowledge. Specifically, for example, Patent Document 3 described above exemplifies each of an aqueous glycerin solution and vitamin E as a single "wet protective agent", but neither exemplifies nor suggests a combination thereof. In addition, Patent Document 3 described above discloses that blood compatibility requires the ability of the "wet protective agent" to scavenge radical species. Even those skilled in the art would never conceive that an aqueous glycerin solution rather inferior in such scavenging ability or water originally having no scavenging ability is added alone to a hollow fiber membrane containing vitamin E already having the sufficient ability to

scavenge radical species.

The hollow fiber membrane containing the particular amount of the fat-soluble vitamin and constituting the hollow fiber membrane-based blood purification apparatus of the present embodiment produces high blood compatibility by covering with an aqueous hydrophilic compound solution and further radiation sterilization. This is not due to only the ability of the fat-soluble vitamin or the aqueous hydrophilic compound solution to scavenge radical species.

The mechanism underlying the excellent blood compatibility exhibited by the hollow fiber membrane-based blood purification apparatus of the present embodiment will be described below.

As shown in Figure 1, the surface of a blend membrane of the polysulfone and the hydrophilic polymer constituting the hollow fiber membrane is constituted such that a hydrophilic polymer 2 is present to protrude from a hydrophobic polysulfone membrane base material 1.

Next, Figure 2 shows a model diagram of a hollow fiber membrane containing no fat-soluble vitamin. Figure 3 shows a model diagram of a hollow fiber membrane containing a fat-soluble vitamin.

As shown in Figure 3, the fat-soluble vitamin, which is hydrophobic, forms an oil layer 5 on the surface of the membrane base material.

An aqueous hydrophilic compound solution added thereto is selectively attached to the hydrophilic polymer 2 also present in the vicinity of the membrane surface, by virtue of the oil layer 5 composed of the hydrophobic fat-soluble vitamin. As shown in Figure 3, a hydrophilic polymer 6 thus formed carries a larger amount of an aqueous hydrophilic compound solution 3 than that in Figure 2.

In the absence of the fat-soluble vitamin, as shown in Figure 2, the aqueous hydrophilic compound solution 3 is also retained by the polysulfone membrane base material 1. In this case, the hydrophilic polymer 2 carries the aqueous hydrophilic compound solution 3 in a lower amount than that by the hydrophilic polymer 6 carrying the aqueous hydrophilic compound solution shown in Figure 3.

Specifically, the combined use of the aqueous hydrophilic compound solution 3 and the fat-soluble vitamin 5 can increase the amount of the aqueous hydrophilic compound solution carried by the hydrophilic polymer 2, compared with the use of the aqueous hydrophilic compound solution alone. This can effectively prevent the deterioration of the hydrophilic polymer 2. In addition, this can enhance wettability at the initial stage after start of use and is consequently effective in improving affinity for blood.

Thus, when the hollow fiber membrane containing no fat-soluble vitamin 5 was covered with an aqueous hydrophilic compound solution, the solution was insufficiently carried by the hydrophilic polymer 2, probably leading to insufficient protective effect against radical species. This idea can consistently explain the findings of the present inventors.

(Amount of fat-soluble vitamin on the surface of the hollow fiber membrane)

In the hollow fiber membrane-based blood purification apparatus of the present embodiment, the amount of the fat-soluble vitamin of the surface of the hollow fiber membrane is 1.5 mg or more and 23 mg or less based on 1 g of the hollow fiber membrane.

If the amount of the fat-soluble vitamin on the surface of the hollow fiber membrane is smaller than 0.5 mg, favorable blood compatibility cannot be obtained due to the deterioration of the hydrophilic polymer during radiation sterilization or difficult control of crosslinking reaction. Accordingly, the amount of the fat-soluble vitamin on the surface of the hollow fiber membrane needs to be 1.5 mg or larger.

If the amount of the fat-soluble vitamin on the surface of the hollow fiber membrane exceeds 25 mg, the increased influence of hydrophobicity of the fat-soluble vitamin itself reduces antithrombotic properties. Upon contact with blood, the resulting hollow fiber membrane may coagulate blood within the hollow fibers and thereby cause so-called residual blood. Accordingly, the amount of the fat-soluble vitamin on the surface of the hollow fiber membrane is 23 mg or smaller, preferably 18 mg or smaller, in terms of the upper limit.

The amount of the fat-soluble vitamin present on the surface of the hollow fiber membrane constituting the hollow fiber membrane-based blood purification apparatus of the present embodiment can be evaluated as follows: the fat-soluble vitamin on the surface of the hollow fiber membrane is extracted with an aqueous alcohol solution and then quantified by liquid chromatography.

In the present specification, the surface of the hollow fiber membrane includes all of the inner and outer surfaces of the hollow fiber membrane and the surfaces of pores in the porous portion in the thickness portion of the membrane.

An example of a specific method for measuring the amount of the fat-soluble vitamin present on the surface of the hollow fiber membrane constituting the hollow fiber membrane-based blood purification apparatus will be shown below. The method for measuring the amount of the fat-soluble vitamin is not intended to be limited to the example shown below and can be adjusted appropriately according to the amount of the fat-soluble vitamin collected, the concentration or amount of an extract, temperature, time, a solution flow rate, a measurement apparatus, etc.

First, the hollow fiber membrane-based blood purification apparatus is disassembled to collect the hollow fiber

membrane, which is in turn washed with water and then dried.

Subsequently, 4 g, for example, of the hollow fiber membrane thus dried is weighed into a glass bottle. After addition of, for example, 80 mL of 75 v/v% aqueous ethanol solution, the fat-soluble vitamin is extracted at room temperature for 60 minutes with ultrasonic vibration applied thereto.

Next, liquid chromatography is performed to determine the amount of the fat-soluble vitamin in the extract using a calibration curve obtained from the peak area of a fat-soluble vitamin standard solution. For example, a column (manufactured by Shodex Asahipak, ODP-506E packed column for HPLC) is attached to a high-performance liquid chromatography apparatus (pump: JASCO PU-1580, detector: Shimadzu RID-6A, autoinjector: Shimadzu SIL-6B, data processing: Tosoh GPC-8020, column oven: GL Sciences 556). Methanol for high-performance liquid chromatography is applied as a mobile phase to the column of 40°C at a flow rate of, for example, 1 mL/min. The fat-soluble vitamin concentration is determined from the absorption peak area of the UV portion. From this concentration, the weight (mg/g) of the fat-soluble vitamin present on the surface of the hollow fiber membrane is determined based on 1 g of the hollow fiber membrane with extraction efficiency as 100%.

The radiation sterilization partially inactivates the fat-soluble vitamin.

The " amount of the fat-soluble vitamin in the surface of the hollow fiber membrane" in the hollow fiber membrane-based blood purification apparatus of the present embodiment is defined as the amount of fat-soluble vitamins on the surface of the hollow fiber membrane, including fat-soluble vitamins inactivated due to radiation sterilization.

By contrast, the "antioxidative capacity" described later is evaluated with the amount of active fat-soluble vitamins (i.e., fat-soluble vitamins not inactivated by radiation sterilization) on the surface of the hollow fiber membrane as an index.

(Attachment rate of aqueous hydrophilic compound solution covering hollow fiber membrane)

The hollow fiber membrane constituting the hollow fiber membrane-based blood purification apparatus of the present embodiment is covered with an aqueous hydrophilic compound solution having a 60 % with respect to its dry weight to a saturated liquid content (inclusive).

When the hollow fiber membrane is covered with an aqueous hydrophilic compound solution, it is preferred that the aqueous hydrophilic compound solution should be attached thereto so that it can be washed off easily in use at hospital.

In this context, the washing off means that at least 95% or more of the aqueous hydrophilic compound solution covering the hollow fiber membrane is washed off by general priming operation performed before use to reproduce the surface of the hollow fiber membrane.

Examples of the aqueous hydrophilic compound solution covering the hollow fiber membrane may include solutions containing a polyhydric alcohol (e.g., glycerin, mannitol, and glycols) dissolved in water or a physiological solution. These aqueous hydrophilic compound solutions can uniformly cover the surface of the hollow fiber membrane and can be washed off easily.

An aqueous solution of a polyhydric alcohol is preferable from the viewpoint of protecting the hydrophilic polymer from radical species. Particularly, an aqueous solution of glycerin or polyethylene glycol is more preferable in terms of its proved use as a pore size keeping agent or surface modifier for hollow fiber membranes. An aqueous glycerin solution is even more preferable.

The aqueous hydrophilic compound solution may be mixed with the predetermined additive.

Examples of the additive may include: water-soluble vitamins such as vitamin C (and its derivatives and salts such as tocopherol acetate and $\alpha$-tocotrienol); and sugars such as glucose, mannose, xylose, ribose, fructose, and trehalose.

As described above, the hollow fiber membrane in the hollow fiber membrane-based blood purification apparatus of the present embodiment is covered with an aqueous hydrophilic compound solution having a 60% to a saturated liquid content (inclusive) with respect to its dry weight.

The saturated liquid content is on the order of 400% for hollow fiber membranes having general water permeability of 200 mL/hr·mmHgm$^2$ (1 mmHg = 133.32 Pa) as hemodialysis membranes. Thus, examples below will be described with the saturated liquid content as 400%. However, the hollow fiber membrane-based blood purification apparatus of the present embodiment is not intended to be limited to the examples below. In this context, it is to be understood that the "saturated liquid content" means the maximum liquid content at which the aqueous hydrophilic compound solution does not drip off.

Specifically, the "saturated liquid content" can be measured by, for example, the following method:

The inside of the hollow fiber membrane-based blood purification apparatus is fully filled with an aqueous hydrophilic compound solution at room temperature. Next, 0.15 MPa compressed air is blown to only the inside of the hollow fibers for 10 seconds to drain water from the hollow portion. In this procedure, a nozzle communicating with the internal space (outside of the hollow fibers) of the blood purification apparatus is stoppered to prevent liquids present

in the thickness portion of the membrane from getting out. Subsequently, compressed air is blown in the same way as above to only the outside of the hollow fibers to drain water from the internal space portion of the blood purification apparatus. In this way, the state in which the aqueous solution is retained in the thickness portion of the hollow fiber membrane and in the gap between the hollow fibers can be reproduced. Its weight can be measured to thereby determine the saturated liquid content.

If the attachment rate of the aqueous hydrophilic compound solution to the hollow fiber membrane of this example exceeds 400%, the resulting hollow fiber membrane-based blood purification apparatus has an excessively large weight and impairs the advantages of dry type. In addition, liquid droplets easily adhere to the inner wall of a container constituting the blood purification apparatus or to the inside of a sterilization bag around room temperature (on the order of 20 to 40°C) at which the blood purification apparatus is generally stored or distributed. The resulting product has poor appearance. Moreover, when hollow fiber membrane bundles are assembled into the apparatus with the attachment rate of the aqueous hydrophilic compound solution adjusted, the hollow fiber membrane easily adheres to another due to sticky outer surface. This hinders the entrance of the potting agent and causes leakage. Furthermore, when the attachment rate of the aqueous hydrophilic compound solution is adjusted after assembly, such adhesion also occurs and may present an obstacle to dialysis efficiency. In addition, radiotransparency is weakened due to the increased density of a portion of the hollow fiber membrane. In this regard, a large radiation for securing the minimum exposure dose necessary for sterilization increases the dose distribution of the hollow fiber membrane-based blood purification apparatus and thereby locally deteriorates the materials, disadvantageously resulting in increase in elution.

Thus, the hollow fiber membrane needs to be covered with an aqueous hydrophilic compound solution having a 60% to the saturated liquid content (inclusive) with respect to its dry weight. The hollow fiber membrane of the example described above needs to be covered with an aqueous hydrophilic compound solution of 400% or less, preferably 350% or less, more preferably 300% or less, in terms of the upper limit with respect to its dry weight.

The attachment rate needs to be 60% or more, more preferably 80% or more, even more preferably 100% or more, in terms of the lower limit from the viewpoint of protecting the hydrophilic polymer constituting the hollow fiber membrane.

The attachment rate of the aqueous hydrophilic compound solution to the hollow fiber membrane is calculated as a ratio of the total weight of the aqueous hydrophilic compound solution to the dry weight of the hollow fiber membrane.

A method for measuring the attachment rate of the aqueous hydrophilic compound solution is not particularly limited, and the attachment rate of the aqueous hydrophilic compound solution can be measured, for example, by the following method:

First, 5 g of the hollow fiber membrane is taken out of the hollow fiber membrane-based blood processing apparatus, and the weight (A) of the hollow fiber membrane before drying is accurately measured.

Then, only water is removed using a vacuum dryer. The weight (B) of the hollow fiber membrane thus dried is measured.

Then, the whole amount of the dried hollow fiber membrane sample from which only water has been removed is chopped. After addition of 300 mL of pure water, the container is stoppered and washed with an ultrasonic washing apparatus for 60 minutes to extract the attached hydrophilic compound, i.e., the polyhydric alcohol.

The polyhydric alcohol is quantified by liquid chromatography using the extract thus obtained using the ultrasonic washing apparatus from the chopped hollow fiber membrane sample. The weight (C) of the polyhydric alcohol in the extract is determined using a calibration curve obtained from the peak area of a standard solution.

Only the chopped hollow fiber membrane sample is further taken out of the extract and dried in a vacuum dryer. Then, the weight of the chopped hollow fiber membrane sample thus dried is measured and used as the weight (D) of the hollow fiber membrane unattached with the polyhydric alcohol and water.

A value calculated according to the formula (I) shown below based on these measured values is defined as an attachment rate of water. A value calculated according to the formula (II) shown below based thereon is defined as an attachment rate of the polyhydric alcohol.

In Examples and Comparative Examples described later, the respective attachment rates of water and the polyhydric alcohol (glycerin, tetraethylene glycol, or polyethylene glycol) were determined according to the formulas (I) and (II), respectively. The attachment rate of the aqueous hydrophilic compound solution was determined as the total thereof.

$$\text{Attachment rate (\%) of water} = [(A - B) / D] \times 100 \quad \ldots \text{(I)}$$

$$\text{Attachment rate (\%) of polyhydric alcohol} = (C / D) \times 100 \quad ... \text{(II)}$$

(Ratio of components in aqueous hydrophilic compound solution covering hollow fiber membrane)

The aqueous hydrophilic compound solution attached to the hollow fiber membrane constituting the hollow fiber membrane-based blood purification apparatus of the present embodiment is an aqueous polyhydric alcohol solution. The weight ratio of the polyhydric alcohol to water is 1.3 times or more and 4.8 times or less.

If the weight ratio of the polyhydric alcohol to water exceeds 7.5 times, the increased local concentration of the polyhydric alcohol attached to the surface or inside of the hollow fiber membrane may make the attachment site viscous, resulting in a nonuniform covering state. In this case, protective effect tends to be rather insufficient against exposure to radiation described later. In addition, a rise in the freezing point of the aqueous polyhydric alcohol solution facilitates the freezing of the aqueous solution contained in the pores of the hollow fiber membrane and thus causes damage accompanied by the structural change of the hollow fiber membrane. Thus, the weight ratio of the polyhydric alcohol to water is preferably 4.8 times or smaller. Particularly, when the polyhydric alcohol is glycerin, the weight ratio is more preferably 4.8 times or smaller at which the aqueous glycerin solution has a freezing point of -10°C or lower, even more preferably 3 times or smaller at which the aqueous glycerin solution has a freezing point of -30°C or lower.

Its lower limit is 1.3 times from the viewpoint of securing practically sufficient protective effect.

Particularly, when the polyhydric alcohol is glycerin, the weight ratio is more preferably 1.3 times or larger at which the aqueous glycerin solution has a freezing point of -10°C or lower, even more preferably 1.3 times or larger at which the aqueous glycerin solution has a freezing point of -30°C or lower.

The weight ratio of the polyhydric alcohol to water is determined according to the following formula (III):

$$\text{Weight ratio of polyhydric alcohol to water} = \text{weight (g) of polyhydric alcohol} / \text{weight (g) of water} \quad ... \text{(III)}$$

(Attachment rate of polyhydric alcohol covering hollow fiber membrane)

The aqueous hydrophilic compound solution covering the hollow fiber membrane in the hollow fiber membrane-based blood purification apparatus of the present embodiment is an aqueous polyhydric alcohol solution. It is preferred that the polyhydric alcohol having a weight ratio of 20% or more and 300% or less with respect to the dry weight of the hollow fiber membrane should be attached to the hollow fiber membrane in the hollow fiber membrane-based blood purification apparatus. When the polyhydric alcohol is attached in the form of an aqueous solution to the hollow fiber membrane, the attachment rate of the polyhydric alcohol to the hollow fiber membrane refers to the ratio of the net weight of the polyhydric alcohol excluding the water component to the dry weight of the hollow fiber membrane and can be calculated according to the formula (II).

If the ratio of the weight of the polyhydric alcohol to the dry weight of the hollow fiber membrane (attachment rate of the polyhydric alcohol) exceeds 300%, the resulting hollow fiber membrane-based blood purification apparatus has a large weight, impairs the advantages of dry type, and is poorly handleable. In addition, liquid droplets more easily adhere to the inner wall of a container constituting the blood purification apparatus or to the inside of a sterilization bag around room temperature (on the order of 20 to 40°C) at which the blood purification apparatus is generally stored or distributed. The resulting product has poor appearance. Furthermore, the increased local concentration of the polyhydric alcohol attached to the surface or inside of the hollow fiber membrane may make the attachment site viscous, resulting in a nonuniform covering state. In this case, protective effect is rather insufficient against exposure to radiation described later.

Thus, the attachment rate of the polyhydric alcohol is preferably 300% or less, more preferably 250% or less, even more preferably 200% or less.

The aqueous hydrophilic compound solution is an aqueous polyhydric alcohol solution. It is preferred that the polyhydric alcohol of 20% or more with respect to the dry weight of the hollow fiber membrane should be attached to the hollow fiber membrane in the hollow fiber membrane-based blood purification apparatus from the viewpoint of securing the practically sufficient protective effect on the hollow fiber membrane.

The low attachment rate of the polyhydric alcohol can lower the density of the whole hollow fiber membrane bundle and thereby reduce radiation dose distribution during radiation sterilization.

This also produces the advantage that the polyhydric alcohol can be removed more quickly and more reliably by priming operation before use. From the viewpoint of securing practically sufficient protective effect against the deterioration of the hollow fiber membrane, the polyhydric alcohol of preferably 20% or more, more preferably 50% or more, even more preferably 80% or more, with respect to the dry weight of hollow fiber membrane is attached thereto, as described above.

(Attachment rate of water covering hollow fiber membrane)

For the hollow fiber membrane constituting the hollow fiber membrane-based blood purification apparatus of the present embodiment, it is preferred that the attachment rate of the polyhydric alcohol should fall within the numerical range described above while the ratio of a water content to the dry weight of the hollow fiber membrane, i.e., the attachment rate of water, should be 40% or more and less than 100%.

When the attachment rate of water is 40% or more with respect to the dry weight of the hollow fiber membrane, platelet activation can be inhibited at the stage of initial contact with blood. This is probably because the hydrophilic polymer becomes hydrated on the moderately wetted surface of the hollow fiber membrane, resulting in enhanced wettability at the initial stage after start of use and improved affinity for blood. This feature is very important when the blood purification apparatus of dry type must be used immediately after priming.

However, if the attachment rate of water is 100% or more with respect to the dry weight of the hollow fiber membrane, water contained in the pores of the hollow fiber membrane may be frozen, particularly under low temperature conditions, even in the absence of water in the neighborhood of the hollow fiber membrane. In this case, damage accompanied by the structural change of the hollow fiber membrane easily occurs. If the attachment rate of water exceeds 100% with respect to the dry weight of the hollow fiber membrane, excessive water forms water droplets, which in turn adhere to the inner wall of a container or to the inside of a sterilization bag and become responsible for the deteriorated appearance of the product.

The ratio of a water content to the dry weight of the hollow fiber membrane (attachment rate of water) is preferably 40% or more. If the attachment rate of water is less than 40%, platelet may be activated at the stage of initial contact with blood to reduce blood compatibility. This is probably because the hydrophilic polymer exhibits reduced molecular mobility on the excessively dried surface of the hollow fiber membrane and therefore requires time for being wetted with water and converted to a hydrated state in use.

Particularly when a polyhydric alcohol is used in the aqueous hydrophilic compound solution, the increased viscosity of the aqueous polyhydric alcohol solution is responsible for greatly varied attachment rates of water to the hollow fiber membrane. The resulting hollow fiber membrane tends to be exceedingly low hydrophilic and consequently cause reduction in blood compatibility. Accordingly, it is preferred that the attachment rate of water of 40% or more, more preferably 60% or more, should be secured.

In the hollow fiber membrane-based blood purification apparatus of the present embodiment, a method for setting the attachment rate of the aqueous hydrophilic compound solution or the respective attachment rates of the polyhydric alcohol and water to the hollow fiber membrane to the ranges described above is not particularly limited.

Examples thereof may include a sequential method involving contacting a high concentration of the aqueous hydrophilic compound solution with the hollow fiber membrane and then flowing water thereto to thereby adjust the respective attachment rates of the aqueous hydrophilic compound solution, the polyhydric alcohol, and water to the predetermined ranges described above. Alternatively, for example, a one-step method involves optimizing the time for which the aqueous hydrophilic compound solution is brought into contact with the hollow fiber membrane, the concentration of this solution, the pressure of injection into the hollow fiber membrane, and air flash conditions for blowing off the aqueous hydrophilic compound solution, etc. The latter process is less complicated and is advantageous in uniform covering because it eliminates the need for using the high concentration of the aqueous solution. The air flash method is particularly effective in blowing off the residues of the aqueous hydrophilic compound solution and is also effective in more uniform covering that achieves, for example, the extension of a viscous aqueous hydrophilic compound solution to the inner surface of the hollow fiber membrane.

The method for setting the attachment rate of the aqueous hydrophilic compound solution or the respective attachment rates of the polyhydric alcohol and water to the hollow fiber membrane to the ranges described above may be performed for each hollow fiber membrane as part of the membrane formation process or may be performed for a hollow fiber membrane bundle obtained after membrane formation.

In the method performed for each hollow fiber membrane, a membrane formation line may be provided with a bath of the aqueous hydrophilic compound solution, in which the hollow fiber membrane is dipped. In the method performed for a hollow fiber membrane bundle obtained after membrane formation, the hollow fiber membrane bundle may be dipped in a bath of the aqueous hydrophilic compound solution or showered with the aqueous hydrophilic compound solution from the end of the hollow fiber membrane bundle.

Other applicable methods involve flowing the aqueous hydrophilic compound solution from the opening of the

hollow fiber membrane in a potting portion in the state of a semifinished product of the hollow fiber membrane-based blood purification apparatus, i.e., in the state unattached to a header during assembly, involves attaching thereto a header having a fluid inlet/outlet for communication with the inside of the hollow fiber membrane and then flowing the solution from the fluid inlet/outlet of the header, or involves flowing the solution from the fluid inlet/outlet (e.g., dialysis solution inlet/outlet for hemodialyzers) of a tubular container. Among them, the method involving flowing the solution from the opening of the hollow fiber membrane in a semifinished product of the blood purification apparatus or flowing the solution from the inlet/outlet of the header in the blood purification apparatus is preferable because even a highly viscous solution can be flowed reliably into the inside of the hollow, which comes in contact with blood.

(Time-dependent stability of antioxidative capacity)

In recent years, blood purification therapy has been used in various regions. Modules will be required to have much higher storage stability for use in diverse environments.

Generally, three-year storage stability under room temperature and normal humidity conditions has allegedly been practically sufficient for conventional modules.

Thus, the present inventors paid attention to, particularly, antioxidative capacity among various properties as the storage stability of the module. Focusing on a new challenge of acquiring the time-dependent stability of antioxidative capacity of the module over long-term storage, the present inventors conducted diligent studies to find the optimum constitution of the hollow fiber membrane in the module. Specifically, in addition to the blood compatibility, a further object of the present invention is to acquire the time-dependent stability of antioxidative capacity of the module over long-term storage.

The time-dependent stability of antioxidative capacity of the module over long-term storage is evaluated by a time-dependent accelerated test. For example, the module can be heat-treated for 3 weeks in an environment of 60°C and then evaluated for its antioxidative capacity.

The fat-soluble antioxidant (fat-soluble vitamin) responsible for the antioxidative capacity of the module is present on the surface of the hollow fiber membrane in the hollow fiber membrane-based blood purification apparatus of the present embodiment. When the aqueous hydrophilic compound solution covering the hollow fiber membrane is an aqueous polyhydric alcohol solution, a blood purification apparatus having a weight ratio of the polyhydric alcohol to water set to equal to or higher than the given ratio can retain effective antioxidative capacity even after time-dependent acceleration treatment.

By most definitions, it is assumed that the fat-soluble antioxidant (fat-soluble vitamin) coexisting with the polyhydric alcohol is first oxidized due to its higher antioxidative capacity to thereby prevent the oxidation of the polyhydric alcohol.

In the hollow fiber membrane-based blood purification apparatus of the present embodiment, however, the fat-soluble antioxidant (fat-soluble vitamin) may exhibit behavior different from the assumption. Specifically, the fat-soluble antioxidant (fat-soluble vitamin) is adsorbed through its hydrophobicity onto the polysulfone (which is a hydrophobic polymer) membrane. As a result, only the aqueous polyhydric alcohol solution comes in contact with outside air. Therefore, oxygen (or active oxygen) is consumed by the oxidation of the aqueous polyhydric alcohol solution before reaching the fat-soluble antioxidant. A module based on such a principle in which oxygen is consumed by the polyhydric alcohol can exert excellent antioxidative properties even under severe conditions. From such viewpoints, the weight ratio of the polyhydric alcohol to water in the aqueous polyhydric alcohol solution is 1.3 times or larger, preferably 2.3 times or larger.

On the other hand, too high a weight ratio of the polyhydric alcohol to water impairs storage stability. Specifically, the polyhydric alcohol itself is converted to a radical while the polyhydric alcohol works as a radical scavenger. This radical is relatively stable (thus useful as a radical scavenger) and consequently lasts long. In other words, a high polyhydric alcohol concentration means the easy accumulation of radicals generated during sterilization or radicals generated by oxygen invasion, heat, light, or the like, accompanied by storage in a severe environment. Particularly, a high polyhydric alcohol concentration facilitates the oxidation of the fat-soluble vitamin and causes the inhibition of the antioxidative properties as described above. From these viewpoints, the weight ratio of the polyhydric alcohol to water is 4.8 times or smaller.

Examples

Hereinafter, the present invention will be described with reference to specific Examples and Comparative Examples thereof.

First, various measurement methods used in Examples will be described.

[Amount of fat-soluble vitamin on the surface of the hollow fiber membrane]

A specific method for measuring the amount of a fat-soluble vitamin on the surface of a hollow fiber membrane will be described.
First, the hollow fiber membrane-based blood purification apparatus was disassembled to collect the hollow fiber membrane, which was in turn washed with water and then dried in vacuum at 40°C.
4 g of the hollow fiber membrane thus dried was weighed into a glass bottle. After addition of 80 mL of 75 v/v% aqueous ethanol solution, the fat-soluble vitamin was extracted at room temperature for 60 minutes with ultrasonic vibration applied thereto.
Quantification operation was performed by liquid chromatography to determine the amount of the fat-soluble vitamin in the extract using a calibration curve obtained from the peak area of a fat-soluble vitamin standard solution.
Specifically, a column (manufactured by Shodex Asahipak, ODP-506E packed column for HPLC) was attached to a high-performance liquid chromatography apparatus (pump: JASCO PU-1580, detector: Shimadzu RID-6A, autoinjector: Shimadzu SIL-6B, data processing: Tosoh GPC-8020, column oven: GL Sciences 556). Methanol for high-performance liquid chromatography was applied as a mobile phase to the column of 40°C at a flow rate of 1 mL/min. The fat-soluble vitamin concentration was determined from the absorption peak area of the UV portion.
From this fat-soluble vitamin concentration, the weight of the fat-soluble vitamin present on the surface of the hollow fiber membrane was determined based on 1 g of the hollow fiber membrane with extraction efficiency as 100% and used as the amount of the fat-soluble vitamin (mg/g).

[Antioxidative capacity]

A specific method for measuring the antioxidative capacity of the hollow fiber membrane-based blood purification apparatus after radiation sterilization will be described.
First, ferric chloride hexahydrate was dissolved in pure water to prepare 0.3 w/v% (amount (g) of solute in 100 mL of solution) aqueous solution.
The hollow fiber membrane-based blood purification apparatus was disassembled to collect the hollow fiber membrane, which was in turn washed with water and then dried in vacuum at 40°C.
Next, 1 g of the hollow fiber membrane thus dried and 20 mL of the aqueous ferric chloride solution were weighed into a glass bottle, degassed at 7.999 kPa (60 mmHg) for 10 minutes, and then incubated at 30°C x 4 hours under shaking (the fat-soluble vitamin present on the surface of the hollow fiber membrane reduced iron (III) ions to form iron (II)).
2.6 mL of the incubated aqueous solution was mixed with 0.7 mL of ethanol and 0.7 mL of 0.5 w/v% aqueous ethanol solution of 2,2'-bipyridyl separately prepared, and the mixture was incubated at 30°C x 30 minutes under shaking (iron (II) and bipyridyl formed a complex to develop color).
The absorbance of the colored solution was measured at 520 nm using a spectrometer.
The same incubation, color reaction, and absorbance measurement as above were performed using an ethanol solution of a fat-soluble vitamin with a known concentration instead of the hollow fiber membrane to prepare a calibration curve. The antioxidative capacity exhibited by 1 g of the hollow fiber membrane was determined as a value based on the weight of active fat-soluble vitamins.
The antioxidative capacity was determined to be favorable (O) when the value based on the weight of active fat-soluble vitamins present on the surface of the hollow fiber membrane was 0.4 mg or higher based on 1 g of the hollow fiber membrane. By contrast, the antioxidative capacity was determined to be practically unfavorable (x) when this value was lower than 0.4 mg.

[Determination of lactate dehydrogenase (LDH) activity]

The blood compatibility of the hollow fiber membrane was evaluated on the basis of platelet attachment to the surface of the hollow fiber membrane and quantified with the activity of lactate dehydrogenase (LDH) contained in the platelet attached to the hollow fiber membrane as an index.
Previous model tests on blood compatibility have also been conducted with lactate dehydrogenase (LDH) as an index. In these tests, however, blood is brought into contact with test samples only for a short time.
In the present determination method, severe conditions were adopted under which blood was brought into contact (circulated) with test samples for a long time to facilitate platelet attachment for the verification of a higher level of blood compatibility.
First, the hollow fiber membrane-based blood purification apparatus was disassembled to collect the hollow fiber membrane, both ends of which were in turn bonded using an epoxy adhesive such that an effective length was 15 cm and the inner surface area of the membrane was 50 $mm^2$ (as an example, 56 filaments of hollow fibers with an inner

diameter of 185 μm) to prepare a mini module.

The mini module was washed with 3 mL of saline (manufactured by Otsuka Pharmaceutical Co., Ltd., Otsuka Seishoku Chu) flowed at a flow rate of 0.6 mL/min in the inside of the hollow fiber membrane (hereinafter, this procedure is referred to as "priming").

Then, 15 mL of heparin-supplemented human blood was adjusted to a temperature of 37°C and circulated at a flow rate of 1.2 mL/min for 4 hours in the mini module. After the circulation, the inside and outside of the mini module were each washed with 10 mL of saline.

From the mini module thus washed, a hollow fiber membrane of 7 cm in length with 56 filaments was collected, then chopped, and placed in a Spitz tube for LDH assay to prepare an assay sample.

Next, Triton X-100 (manufactured by Nacalai Tesque, Inc.) was dissolved in phosphate-buffered saline (PBS) (manufactured by Wako Pure Chemical Industries, Ltd.). 0.5 mL of 0.5% by volume of the obtained Triton X-100/PBS solution was added to the Spitz tube for LDH assay and then centrifuged (2700 rpm x 5 min) to precipitate the hollow fiber membrane in the solution. The cells (mainly, platelet) attached to the hollow fiber membrane were disrupted by extraction for 60 minutes under shaking to extract intracellular LDH. A 0.05 mL aliquot of this extract was collected and reacted by the further addition of 2.7 mL of 0.6 mM sodium pyruvate solution and 0.3 mL of 1.277 mg/mL nicotinamide adenine dinucleotide (NADH) solution. After reaction for at 37°C for additional one hour, the absorbance was measured at 340 nm.

Likewise, the absorbance of a blood-unreacted hollow fiber membrane (blank) was measured, and the difference in absorbance therebetween was calculated according to the formula (IV) shown below.

The value obtained according to the formula (IV) below was divided by an effective membrane area as shown below in the formula (V). The LDH activity was evaluated on the basis of the obtained value.

In this evaluation method, the larger rate of this decrease means higher LDH activity, i.e., the larger amount of the platelet attached to the surface of the hollow fiber membrane.

The hollow fiber membrane was determined to be particularly favorable (⊙) when LDH was 0 or more and less than 10, to be practically favorable (○) when LDH was 10 or more and less than 50, and to be practically unfavorable (×) when LDH was of 50 or higher.

$$\text{Difference in absorbance at 340 nm } (\Delta 340 \text{ nm}) = \text{Absorbance of sample after 60 minutes} - \text{Absorbance of blank after 60 minutes} \quad \ldots \text{(IV)}$$

$$\text{LDH activity} = \Delta 340 \text{ nm} / \text{Effective area of hollow fiber membrane} \quad \ldots \text{(V)}$$

Figure 4 shows the amount of the fat-soluble vitamin on the surface, i.e., the relationship between the amount of the fat-soluble vitamin present on the surface of the hollow fiber membrane based on 1 g of the hollow fiber membrane and the LDH activity.

In Figure 4, the unit (mg/gHF) indicated in the abscissa means the amount of the fat-soluble vitamin present on the surface of the hollow fiber membrane based on 1 g of the hollow fiber membrane.

It was demonstrated that an amount of fat-soluble vitamin of 0.5 mg or more and 25.0 mg or less based on 1 g of the hollow fiber membrane resulted in favorable LDH, whereas an amount of 0.4 mg or 26.2 mg that fell outside this range resulted in unfavorable LDH.

[Product appearance]

Product appearance was evaluated by the visual observation of the presence or absence of liquid droplets of an aqueous hydrophilic compound solution within a container and a product bag.

Specifically, 50 products were evaluated and determined to be favorable when no liquid droplet was confirmed.

[Model test on high-temperature or long-term storage (time-dependent accelerated test)]

Hollow fiber membrane-based blood purification apparatuses of Examples and Comparative Examples described later were separately heat-treated for 3 weeks in a thermostat bath of 60°C (time-dependent accelerated test).

The antioxidative capacity of each hollow fiber membrane-based blood purification apparatus thus heat-treated was evaluated by the measurement method described above in the paragraph [Antioxidative capacity].

[Example 1]

17 parts by mass of PSf (manufactured by Solvay Advanced Polymers, P-1700)
4 parts by mass of PVP (manufactured by ISP Ltd., K-90)
0.5 parts by mass of α-tocopherol
79 parts by mass of dimethylacetamide (hereinafter, referred to as DMAC)

These components were used to prepare a uniform dope.
In this context, PSf represents a polysulfone-based resin, and PVP represents polyvinylpyrrolidone.
In Example 1, the fat-soluble vitamin (α-tocopherol) was added to a hollow fiber membrane by means of blending.
42% aqueous solution of DMAC was used as a coagulating solution and discharged from a spinneret together with the dope.
The amounts of the dope and the coagulating solution discharged were adjusted such that the membrane thickness and the inner diameter were 45 μm and 185 μm, respectively, after drying.
The discharged dope was dipped in a coagulation bath (containing water) of 60°C positioned 50 cm below the spinneret. After a coagulation step at a rate of 30 m/min and a washing step using water, the hollow fiber membrane was introduced to a dryer, dried at 120°C for 2 minutes, and further heat-treated at 160°C for 0.5 minutes. Then, the crimped polysulfone-containing hollow fiber membrane was wound up.
The wound-up hollow fiber membrane bundle of 10000 filaments was loaded in a plastic tubular container designed such that the hollow fiber membrane had an effective membrane area of 1.5 $m^2$. Both ends thereof were fixed by bonding with a urethane resin. The surfaces at both ends were cut so that the hollow portion of the hollow fiber membrane was open at both ends.
From the open end, an aqueous solution of glycerin (manufactured by Wako Pure Chemical Industries, Ltd., special grade) with a concentration of 65.4% was injected into the hollow fiber membrane for 4.3 seconds. After flash with 0.3 MPa air for 10 seconds, both ends were capped with a header.
A nozzle was stoppered on the blood influx/efflux side. The resulting module was sterilized by exposure to 20 kGy electron beam to obtain a hollow fiber membrane-based blood purification apparatus having an effective membrane area of 1.5 $m^2$.
In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 189.1% and 64.5%, respectively.
Results of assaying its properties are shown in Table 1 below.
In the descriptions of Table 1 below, the measured values were rounded off to the nearest whole number.

[Example 2]

17 parts by mass of PSf (manufactured by Solvay Advanced Polymers, P-1700)
4 parts by mass of PVP (manufactured by ISP Ltd., K-90)
79 parts by mass of dimethylacetamide (hereinafter, referred to as DMAC)

These components were used to prepare a uniform dope.
42% aqueous solution of DMAC was used as a coagulating solution and discharged from a spinneret together with the dope.
The amounts of the dope and the coagulating solution discharged were adjusted such that the membrane thickness and the inner diameter were 45 μm and 185 μm, respectively, after drying.
The discharged dope was dipped in a coagulation bath (containing water) of 60°C positioned 50 cm below the spinneret. After a coagulation step at a rate of 30 m/min and a washing step using water, the hollow fiber membrane was introduced to a dryer, dried at 120°C for 2 minutes, and further heat-treated at 160°C for 0.5 minutes. Then, the crimped polysulfone-containing hollow fiber membrane was wound up.
The wound-up hollow fiber membrane bundle of 10000 filaments was loaded in a plastic tubular container designed such that the hollow fiber membrane had an effective membrane area of 1.5 $m^2$. Both ends thereof were fixed

by bonding with a urethane resin. The surfaces at both ends were cut so that the hollow portion of the hollow fiber membrane was open at both ends.

Next, a covering solution containing 0.23% by mass of α-tocopherol dissolved in an aqueous solution of 57% by mass of isopropanol was flowed for 52 seconds from the blood influx nozzle of the blood purification apparatus into the luminal portion of the hollow fiber membrane to contact α-tocopherol therewith.

In Example 2, the fat-soluble vitamin (α-tocopherol) was added to the hollow fiber membrane by means of coating.

The solution remaining in the luminal portion was removed by air flash. Then, the solvent was dried off by the circulation of dry air of 24°C for 30 minutes to cover the membrane with α-tocopherol.

From the open end, an aqueous solution of glycerin (manufactured by Wako Pure Chemical Industries, Ltd., special grade) with a concentration of 63.1% was further injected into the hollow fiber membrane for 4.4 seconds. After flash with 0.3 MPa air for 10 seconds, both ends were capped with a header.

A nozzle was stoppered on the blood influx/efflux side. The resulting module was exposed to 20 kGy electron beam to obtain a hollow fiber membrane-based blood purification apparatus having an effective membrane area of 1.5 $m^2$.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 190.5% and 66.3%, respectively.

Results of assaying its properties are shown in Table 1 below.

In the descriptions of Table 1 below, the measured values were rounded off to the nearest whole number.

[Example 3]

A hollow fiber membrane-based blood purification apparatus was obtained under the same conditions as in Example 1 except that:

0.3 parts by mass of α-tocopherol was used; drying after washing with water was performed at 120°C for 2 minutes; and the subsequent heat treatment was performed at 120°C for 0.5 minutes.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 178.4% and 79.3%, respectively.

Results of assaying its properties are shown in Table 1 below.

In the descriptions of Table 1 below, the measured values were rounded off to the nearest whole number.

[Example 4]

A hollow fiber membrane-based blood purification apparatus was obtained under the same conditions as in Example 1 except that:

1.0 part by mass of α-tocopherol was used; drying after washing with water was performed at 120°C for 2 minutes; and the subsequent heat treatment was performed at 170°C for 0.5 minutes.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 165.5% and 79.3%, respectively.

Results of assaying its properties are shown in Table 1 below.

In the descriptions of Table 1 below, the measured values were rounded off to the nearest whole number.

[Example 5]

A hollow fiber membrane-based blood purification apparatus was obtained under the same conditions as in Example 1 except that:

1.5 parts by mass of α-tocopherol was used; drying after washing with water was performed at 120°C for 2 minutes; and the subsequent heat treatment was performed at 170°C for 0.5 minutes.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 166.9% and 80.2%, respectively.

Results of assaying its properties are shown in Table 1 below.

In the descriptions of Table 1 below, the measured values were rounded off to the nearest whole number.

[Example 6] (not according to the invention)

A hollow fiber membrane-based blood purification apparatus was obtained under the same conditions as in Example 1 except that:

2.5 parts by mass of α-tocopherol was used; drying after washing with water was performed at 120°C for 2 minutes; and the subsequent heat treatment was performed at 180°C for 0.5 minutes.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 166.2% and 79.9%, respectively.

Results of assaying its properties are shown in Table 1 below.

In the descriptions of Table 1 below, the measured values were rounded off to the nearest whole number.

[Example 7]

A hollow fiber membrane-based blood purification apparatus was obtained under the same conditions as in Example 1 except that:

an aqueous solution of glycerin (manufactured by Wako Pure Chemical Industries, Ltd., special grade) with a concentration of 23.1% was injected into the hollow fiber membrane for 4.5 seconds.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 20.0% and 40.0%, respectively.

Results of assaying its properties are shown in Table 1 below.

In the descriptions of Table 1 below, the measured values were rounded off to the nearest whole number.

[Example 8]

A hollow fiber membrane-based blood purification apparatus was obtained under the same conditions as in Example 1 except that:

an aqueous solution of glycerin (manufactured by Wako Pure Chemical Industries, Ltd., special grade) with a concentration of 69.9% was injected into the hollow fiber membrane for 8.2 seconds.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 299.6% and 100.4%, respectively.

Results of assaying its properties are shown in Table 1 below.

In the descriptions of Table 1 below, the measured values were rounded off to the nearest whole number.

[Example 9]

A hollow fiber membrane-based blood purification apparatus was obtained under the same conditions as in Example 1 except that:

an aqueous solution of glycerin (manufactured by Wako Pure Chemical Industries, Ltd., special grade) with a concentration of 83.2% was injected into the hollow fiber membrane for 8.2 seconds.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 290.4% and 45.4%, respectively.

Results of assaying its properties are shown in Table 1 below.

In the descriptions of Table 1 below, the measured values were rounded off to the nearest whole number.

[Example 10]

A hollow fiber membrane-based blood purification apparatus was obtained under the same conditions as in Example 1 except that:

an aqueous solution of polyethylene glycol (manufactured by Katayama Chemical, Ltd., first grade, PEG600) with a concentration of 64.2% was injected into the hollow fiber membrane for 4.6 seconds.

In this hollow fiber membrane-based blood purification apparatus, the saturated liquid content of the hollow fiber membrane was 400%, and the respective attachment rates of the polyhydric alcohol polyethylene glycol and water in the aqueous hydrophilic compound solution were 153.1% and 66.1%, respectively.
Results of assaying its properties are shown in Table 1 below.
In the descriptions of Table 1 below, the measured values were rounded off to the nearest whole number.

[Example 11]

A hollow fiber membrane-based blood purification apparatus was obtained under the same conditions as in Example 1 except that:

an aqueous solution of tetraethylene glycol(manufactured by Wako Pure Chemical Industries, Ltd., Tetraethyleneglycol, 99%) with a concentration of 61.8% was injected into the hollow fiber membrane for 4.2 seconds.

In this hollow fiber membrane-based blood purification apparatus, the saturated liquid content of the hollow fiber membrane was 400%, and the respective attachment rates of the polyhydric alcohol tetraethylene glycol and water in the aqueous hydrophilic compound solution were 150.5% and 67.2%, respectively.
Results of assaying its properties are shown in Table 1 below.
In the descriptions of Table 1 below, the measured values were rounded off to the nearest whole number.

[Example 12]

A hollow fiber membrane-based blood purification apparatus was obtained under the same conditions as in Example 1 except that:

injectable water was injected into the hollow fiber membrane for 7.6 seconds.

In this hollow fiber membrane-based blood purification apparatus, the saturated liquid content of the hollow fiber membrane was 400%, and the attachment rate of water in the aqueous hydrophilic compound solution to this hollow fiber membrane-based blood processing apparatus was 378.4%.
Results of assaying its properties are shown in Table 1 below.
In the descriptions of Table 1 below, the measured values were rounded off to the nearest whole number.

[Example 13]

A hollow fiber membrane-based blood purification apparatus was obtained under the same conditions as in Example 1 except that:

an aqueous solution of glycerin (manufactured by Wako Pure Chemical Industries, Ltd., special grade) with a concentration of 9.3% was injected into the hollow fiber membrane for 4.6 seconds.

In this hollow fiber membrane-based blood purification apparatus, the saturated liquid content of the hollow fiber membrane was 400%, and the respective attachment rates of glycerin and water in the aqueous hydrophilic compound solution were 25.2% and 220.3%, respectively.
Results of assaying its properties are shown in Table 1 below.
In the descriptions of Table 1 below, the measured values were rounded off to the nearest whole number.

[Example 14]

A hollow fiber membrane-based blood purification apparatus was obtained under the same conditions as in Example 1 except that:

0.5 parts by mass of α-tocopherol was used; drying after washing with water was performed under conditions of 120°C for 2 minutes; and heat treatment subsequent to the drying was performed at 130°C for 0.5 minutes.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 173% and 75%, respectively.
Results of assaying its properties are shown in Tables 1 and 3 below.

[Example 15]

A hollow fiber membrane-based blood purification apparatus was obtained under the same conditions as in Example 14 except that:

an aqueous solution of glycerin (manufactured by Wako Pure Chemical Industries, Ltd., special grade) with a concentration of 55% was injected into the hollow fiber membrane for 4.6 seconds.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 145% and 110%, respectively.
Results of assaying its properties are shown in Table 3 below.

[Example 16]

A hollow fiber membrane-based blood purification apparatus was obtained under the same conditions as in Example 1 except that: the exposure dose of electron beam was changed to 15 kGy.
In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 190.5% and 66.3%, respectively.
Results of assaying its properties are shown in Tables 3 and 5 below.

[Example 17]

A hollow fiber membrane-based blood purification apparatus was obtained under the same conditions as in Example 1 except that: the exposure dose of electron beam was changed to 50 kGy.
In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 190.5% and 66.3%, respectively.
Results of assaying its properties are shown in Table 5 below.

[Example 18]

A hollow fiber membrane-based blood purification apparatus was obtained under the same conditions as in Example 1 except that: 25 kGy gamma rays were used in the exposure instead of electron beam.
In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 190.5% and 66.3%, respectively.
Results of assaying its properties are shown in Table 5 below.

[Comparative Example 1]

A hollow fiber membrane-based blood purification apparatus was obtained under the same conditions as in Example 1 except that:

0.2 parts by mass of $\alpha$-tocopherol was used; drying after washing with water was performed at 120°C for 2 minutes; and the subsequent heat treatment was performed at 120°C for 0.5 minutes.

In this hollow fiber membrane-based blood processing apparatus, the respective attachment rates of glycerin and water were 177.9% and 80.3%, respectively.
Results of assaying its properties are shown in Table 2 below.
In the descriptions of Table 2 below, the measured values were rounded off to the nearest whole number.
Favorable blood compatibility was not obtained due to high LDH activity.

[Comparative Example 2]

A hollow fiber membrane-based blood purification apparatus was obtained under the same conditions as in Example 1 except that:

3.0 parts by mass of α-tocopherol was used; drying after washing with water was performed at 120°C for 2 minutes; and the subsequent heat treatment was performed at 180°C for 0.5 minutes.

In this hollow fiber membrane-based blood processing apparatus, the respective attachment rates of glycerin and water were 166.8% and 81.9%, respectively.
Results of assaying its properties are shown in Table 2 below.
In the descriptions of Table 2 below, the measured values were rounded off to the nearest whole number.
Favorable blood compatibility was not obtained due to high LDH activity.

[Comparative Example 3]

A hollow fiber membrane-based blood purification apparatus was obtained under the same conditions as in Example 1 except that:

an aqueous solution of glycerin (manufactured by Wako Pure Chemical Industries, Ltd., special grade) with a concentration of 67.2% was injected into the hollow fiber membrane for 10.3 seconds.

In this hollow fiber membrane-based blood purification apparatus, the saturated liquid content of the hollow fiber membrane was 400%, and the respective attachment rates of glycerin and water in the aqueous hydrophilic compound solution were 310.5% and 105.3%, respectively.
These measured values are shown in Table 2 below.
In the descriptions of Table 2 below, the measured values were rounded off to the nearest whole number.
In this Comparative Example, liquids oozed into between the hollow fiber membranes. The resulting hollow fiber membrane-based blood purification apparatus did not satisfy the requirements for dry type. Thus, assay on its properties was canceled.

[Comparative Example 4]

A hollow fiber membrane-based blood purification apparatus was obtained under the same conditions as in Example 1 except that:

an aqueous solution of glycerin (manufactured by Wako Pure Chemical Industries, Ltd., special grade) with a concentration of 91.2% was injected into the hollow fiber membrane for 8.9 seconds.

In this hollow fiber membrane-based blood purification apparatus, the saturated liquid content of the hollow fiber membrane was 400%, and the respective attachment rates of glycerin and water in the aqueous hydrophilic compound solution were 390.2% and 45.5%, respectively.
These measured values are shown in Table 2 below.
In the descriptions of Table 2 below, the measured values were rounded off to the nearest whole number.
In this Comparative Example, liquids oozed into between the hollow fiber membranes. The resulting hollow fiber membrane-based blood purification apparatus did not satisfy the requirements for dry type. Thus, assay on its properties was canceled.

[Comparative Example 5]

A hollow fiber membrane-based blood purification apparatus was obtained under the same conditions as in Example 1 except that:

the fat-soluble vitamin α-tocopherol was not added into the dope.

In this hollow fiber membrane-based blood processing apparatus, the respective attachment rates of glycerin and water were 180.5% and 66.6%, respectively.
Results of assaying its properties are shown in Table 2 below. In the descriptions of Table 2 below, the measured

values were rounded off to the nearest whole number.

The LDH activity was high. This is because the hollow fiber membrane contained no fat-soluble vitamin and therefore insufficiently retained the hydrophilic polymer solution on its surface.

[Comparative Example 6]

A hollow fiber membrane-based blood purification apparatus was obtained under the same conditions as in Example 1 except that:

the aqueous glycerin solution was not injected.

Results of assaying its properties are shown in Table 2 below.

The LDH activity was high. This is because the hollow fiber membrane did not retain the hydrophilic polymer solution on its surface.

[Example 19]

A hollow fiber membrane-based blood purification apparatus was obtained under the same conditions as in Example 14 except that:

an aqueous solution of glycerin with a concentration of 53% was injected into the hollow fiber membrane for 4.6 seconds.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 136% and 112%, respectively.

Results of assaying its properties are shown in Table 4 below.

The hollow fiber membrane-based blood purification apparatus of Example 19 exhibited practically sufficient properties in terms of usual antioxidative capacity, but was inferior to those of Examples 14 to 16 in the results about antioxidative capacity evaluated by the time-dependent accelerated test.

This is because glycerin was secured in an amount insufficient for the protection of the fat-soluble vitamin in a severe environment, due to the small weight ratio of glycerin to water in the glycerin solution covering the hollow fiber membrane.

[Example 20]

A hollow fiber membrane-based blood purification apparatus was obtained under the same conditions as in Example 14 except that:

an aqueous solution of glycerin with a concentration of 83.5% was injected into the hollow fiber membrane for 4.6 seconds.

In this hollow fiber membrane-based blood purification apparatus, the saturated liquid content of the hollow fiber membrane was 400%, and the respective attachment rates of the polyhydric alcohol glycerin and water in the aqueous hydrophilic compound solution were 293% and 58%, respectively.

Results of assaying its properties are shown in Table 4 below.

The hollow fiber membrane-based blood purification apparatus of Example 20 was practically sufficient in terms of usual antioxidative capacity, but was inferior to those of Examples 14 to 16 in the results about antioxidative capacity evaluated by the time-dependent accelerated test.

This is because a large amount of glycerin-derived radicals was formed during sterilization by exposure to electron beam and remained to accelerate the oxidation of the fat-soluble vitamin and thereby inactivate it, due to too a large weight ratio of glycerin to water in the glycerin solution covering the hollow fiber membrane.

[Table 1]

| | Example 1 | Example 2 | Example 3 | Example 4 | Example 5 | Example 6* | Example 7 | Example 8 | Example 9 | Example 10 | Example 11 | Example 12 | Example 13 | Example 14 |
|---|---|---|---|---|---|---|---|---|---|---|---|---|---|---|
| Amount of fat-soluble vitamin in surface (mg/gHF) | 5.5 | 5.5 | 0.5 | 10.3 | 18.2 | 25.0 | 5.5 | 5.5 | 5.5 | 5.5 | 5.5 | 5.5 | 5.5 | 1.5 |
| Method for adding fat-soluble vitamin | Blending | Coating | Blending | Blending | Blending | Blending | Blending | Blending | Blending | Blending | Blending | Blending | Blending | Blending |
| Aqueous hydrophilic compound solution | Glycerin/ water | Glycerin/ water | Glycerin/ water | Glycerin/ water | Glycerin/ water | Glycerin/ water | Glycerin/ water | Glycerin/ water | Glycerin/ water | PEG/ water | TEG/ water | Water | Glycerin/ water | Glycerin/ water |
| Attachment rate of aqueous hydrophilic compound solution (%) | 254 | 257 | 258 | 245 | 247 | 246 | 60 | 400 | 336 | 219 | 218 | 378 | 246 | 248 |
| Attachment rate of polyhydric alcohol (%) | 189 | 191 | 178 | 166 | 167 | 166 | 20 | 300 | 290 | 153 | 151 | - | 25 | 173 |
| Attachment rate of water (%) | 65 | 66 | 79 | 79 | 80 | 80 | 40 | 100 | 45 | 66 | 67 | 378 | 220 | 75 |
| Polyhydric alcohol/water | 2.9 | 2.9 | 2.2 | 2.1 | 2.1 | 2.1 | 0.5 | 3.0 | 6.4 | 2.3 | 2.2 | - | 0.1 | 2.3 |
| Antioxidative capacity (after sterilization) (mg/gHF) | ○ 4.2 | ○ 4.1 | ○ 0.4 | ○ 9.6 | ○ 17.1 | ○ 24.2 | 04.7 | ○ 4.9 | ○ 5.2 | ○ 4.9 | ○ 4.8 | ○ 5.0 | ○ 5.0 | ○ 1.2 |
| LDH(△abs/hr/$m^2$) | ◎ 3.2 | ◎ 4.4 | ○ 10.4 | ◎ 6.5 | ◎ 6.9 | ○ 10.6 | ○ 14.5 | ○ 12.3 | ○ 13.4 | ○ 10.1 | ○ 11.1 | ○ 45.5 | ○ 39.1 | ◎ 6.7 |
| Product appearance | Favorable | Favorable | Favorable | Favorable | Favorable | Favorable | Favorable | Favorable | Favorable | Favorable | Favorable | Favorable | Favorable | Favorable |

*not according to the invention

When the weight of the hollow fiber membrane before drying is defined as (A), the weight of the hollow fiber membrane from which only water has been removed by drying is defined as (B), the weight of the polyhydric alcohol extracted from the hollow fiber membrane is defined as (C), and the weight of the hollow fiber membrane unattached with the polyhydric alcohol and water is defined as (D), the "attachment rate (%) of water" and the "attachment rate (%) of polyhydric alcohol" in Table 1 refer to values calculated according to the formulas $[(A - B) / D] \times 100$ and $(C / D) \times 100$, respectively. Also, the "attachment rate (%) of the aqueous hydrophilic compound solution" refers to the total value of the "attachment rate (%) of water" and the "attachment rate (%) of polyhydric alcohol".

Furthermore, TEG represents tetraethylene glycol, and PEG represents polyethylene glycol (the same holds true for Table 2).

[Table 2]

| | Comparative Example 1 | Comparative Example 2 | Comparative Example 3 | Comparative Example 4 | Comparative Example 5 | Comparative Example 6 |
|---|---|---|---|---|---|---|
| Amount of fat-soluble vitamin in surface (mg/gHF) | 0.4 | 26.2 | 5.5 | 5.5 | - | 5.5 |
| Method for adding fat-soluble vitamin | Blending | Blending | Blending | Blending | - | Blending |
| Aqueous hydrophilic compound solution | Glycerin/ water | Glycerin/ water | Glycerin/ water | Glycerin/ water | Glycerin/ water | - |
| Attachment rate of aqueous hydrophilic compound solution (%) | 258.2 | 248.7 | 415.8 | 435.6 | 247.1 | - |
| Attachment rate of polyhydric alcohol (%) | 178 | 167 | 311 | 390 | 181 | - |
| Attachment rate of water (%) | 80 | 82 | 105 | 45 | 67 | - |
| Polyhydric alcohol/water | 2.2 | 2.0 | 2.9 | 8.6 | 2.7 | - |
| Antioxidative capacity (after sterilization) (mg/gHF) | × 0.3 | ○ 25.6 | - | - | - | ○ 5.0 |
| LDH (◿abs/hr/$m^2$) | × 289.1 | × 267.3 | - | - | × 301.6 | × 308.6 |
| Product appearance | Favorable | Favorable | Requirements for dry type were not satisfied due to liquid droplets of glycerin or water droplets confirmed in container or product bag | Requirements for dry type were not satisfied due to liquid droplets of glycerin confirmed in container or product bag | Favorable | Favorable |

[Table 3]

| | Example 14 | Example 15 | Example 16 |
|---|---|---|---|
| Amount of fat-soluble vitamin in surface (mg/gHF) | 1.5 | 1.5 | 1.5 |
| Polyhydric alcohol/water | 2.3 | 1.3 | 4.8 |
| Antioxidative capacity after time-dependent acceleration (mg/gHF) | ○ 0.5 | ○ 0.4 | ○ 0.5 |

[Table 4]

| | Example 19 | Example 20 |
|---|---|---|
| Amount of fat-soluble vitamin in surface (mg/gHF) | 1.5 | 1.5 |
| Polyhydric alcohol/water | 1.2 | 5.0 |
| Antioxidative capacity after time-dependent acceleration (mg/gHF) | × 0.3 | × 0.3 |

[Table 5]

| | Example 16 | Example 17 | Example 18 |
|---|---|---|---|
| Radiation species | Electron beam | Electron beam | Gamma rays |
| Dose (kGy) | 15 | 50 | 25 |
| Antioxidative capacity (after sterilization) (mg/gHF) | ○ 4.2 | ○ 4.0 | ○ 4.2 |
| LDH(◿abs/hr/$m^2$) | ◎ 3.4 | ◎ 3.8 | ◎ 3.2 |
| Product appearance | Favorable | Favorable | Favorable |

In Tables 1, 2, 3, 4, and 5, the unit "mg/gHF" for the amount of the fat-soluble vitamin on the surface of the hollow fiber membrane means the weight (mg) of the fat-soluble vitamin on the surface per g of the hollow fiber membrane to be assayed.

As shown in Table 1, it was demonstrated that all the hollow fiber membrane-based blood purification apparatuses of Examples 1 to 14 had practically favorable antioxidative properties and blood compatibility.

As shown in Table 3, it was further demonstrated that the hollow fiber membrane-based blood purification apparatus of the present invention showed excellent storage properties, i.e., high antioxidative capacity, even in a more severe environment by setting the weight ratio of the polyhydric alcohol to water to the range of 1.3 times or more and 4.8 times or less in the aqueous hydrophilic compound solution attached to the hollow fiber membrane.

Industrial Applicability

A hollow fiber membrane-based blood purification apparatus of the present invention has excellent antioxidative properties and blood compatibility and as such, has industrial applicability as a blood purification apparatus used in extracorporeal blood circulation therapy.

Reference Signs List

1 PSf membrane
2 Hydrophilic polymer
3 Aqueous hydrophilic compound solution
4 Hydrophilic polymer carrying an aqueous hydrophilic compound solution
5 Oil layer of a fat-soluble vitamin
6 Hydrophilic polymer carrying a larger amount of an aqueous hydrophilic compound solution

## Claims

1. A hollow fiber membrane-based blood purification apparatus comprising a hollow fiber membrane containing a polysulfone-based resin, a hydrophilic polymer, and a fat-soluble vitamin, the hollow fiber membrane-based blood purification apparatus being radiation-sterilized, wherein
the amount of the fat-soluble vitamin on the surface of the hollow fiber membrane is 1.5 mg or more and 23 mg or less, based on 1 g of the hollow fiber membrane, wherein the amount of the fat-soluble vitamin is determined as described in the description, wherein the surface of the hollow fiber membrane includes the inner and outer surfaces of the hollow fiber membrane, and
the surface of the hollow fiber membrane is covered with a mixture of water and a polyhydric alcohol at a content of 60 % or more, based on the dry weight of the hollow fiber membrane, to the saturated liquid content or less, **characterized in that** the weight ratio of the polyhydric alcohol to water in the mixture is 1.3 times or more and 4.8 times or less.

2. The hollow fiber membrane-based blood purification apparatus according to claim 1, wherein the amount of the fat-soluble vitamin on the surface of the hollow fiber membrane is 1.5 mg or more and 18 mg or less, based on 1 g of the hollow fiber membrane.

3. The hollow fiber membrane-based blood purification apparatus according to claim 1, wherein the attachment rate of water constituting the mixture of water and polyhydric alcohol to the hollow fiber membrane is 40 % or more and less than 100 %, based on the dry weight of the hollow fiber membrane, and the attachment rate of the polyhydric alcohol to the hollow fiber membrane is 20 % or more and 300 % or less, based on the dry weight of the hollow fiber membrane, wherein the attachment rates of water and the polyhydric alcohol are determined as described in the description.

## Patentansprüche

1. Blutreinigungsvorrichtung auf Hohlfasermembranbasis, umfassend eine Hohlfasermembran, die ein Harz auf Polysulfonbasis, ein hydrophiles Polymer und ein fettlösliches Vitamin enthält, wobei die Blutreinigungsvorrichtung auf Hohlfasermembranbasis strahlungssterilisiert ist, wobei die Menge des fettlöslichen Vitamins auf der Oberfläche der Hohlfasermembran 1,5 mg oder mehr und 23 mg oder weniger beträgt, bezogen auf 1 g der Hohlfasermembran, wobei die Menge des fettlöslichen Vitamins so bestimmt wird, wie es in der Beschreibung beschrieben ist, wobei die Oberfläche der Hohlfasermembran die innere und die äußere Oberfläche der Hohlfasermembran umfasst und die Oberfläche der Hohlfasermembran mit einem Gemisch aus Wasser und einem mehrwertigen Alkohol mit einem Gehalt von 60% oder mehr, bezogen auf das Trockengewicht der Hohlfasermembran, bis zum gesättigten Flüssigkeitsgehalt oder weniger bedeckt ist, **dadurch gekennzeichnet, dass** das Gewichtsverhältnis des mehrwertigen Alkohols zu Wasser in dem Gemisch 1,3 oder mehr und 4,8 oder weniger beträgt.

2. Blutreinigungsvorrichtung auf Hohlfasermembranbasis gemäß Anspruch 1, wobei die Menge des fettlöslichen Vitamins auf der Oberfläche der Hohlfasermembran 1,5 mg oder mehr und 18 mg oder weniger beträgt, bezogen auf 1 g der Hohlfasermembran.

3. Blutreinigungsvorrichtung auf Hohlfasermembranbasis gemäß Anspruch 1, wobei die Bindungsrate von Wasser, das das Gemisch aus Wasser und dem mehrwertigen Alkohol bildet, an der Hohlfasermembran 40% oder mehr und weniger als 100% beträgt, bezogen auf das Trockengewicht der Hohlfasermembran, und die Bindungsrate des mehrwertigen Alkohols an der Hohlfasermembran 20% oder mehr und 300% oder weniger beträgt, bezogen auf das Trockengewicht der Hohlfasermembran, wobei die Bindungsraten des Wassers und des mehrwertigen Alkohols so bestimmt werden, wie es in der Beschreibung beschrieben ist.

## Revendications

1. Dispositif de purification de sang basé sur une membrane à fibres creuses comprenant une membrane à fibres creuses contenant une résine à base de polysulfone, un polymère hydrophile, et une vitamine soluble dans une matière grasse, le dispositif de purification de sang basé sur une membrane à fibres creuses étant stérilisé par rayonnement, dans lequel
la quantité de la vitamine soluble dans une matière grasse sur la surface de la membrane à fibres creuses est

supérieure ou égale à 1,5 mg et inférieure ou égale à 23 mg, par rapport à 1 g de la membrane à fibres creuses, dans lequel la quantité de la vitamine soluble dans une matière grasse est déterminée comme décrit dans la description, dans lequel la surface de la membrane à fibres creuses comprend des surfaces intérieure ou extérieure de la membrane à fibres creuses, et

la surface de la membrane à fibres creuses est recouverte avec un mélange d'eau et d'un alcool polyhydrique à une teneur supérieure ou égale à 60 %, par rapport au poids sec de la membrane à fibres creuses, et inférieure ou égale à la teneur en liquide saturé, **caractérisé en ce que** le rapport pondéral de l'alcool polyhydrique à l'eau dans le mélange est supérieur ou égal à 1,3 fois et inférieur ou égal à 4,8 fois.

**2.** Dispositif de purification de sang basé sur une membrane à fibres creuses selon la revendication 1, dans lequel la quantité de la vitamine soluble dans une matière grasse sur la surface de la membrane à fibres creuses est supérieure ou égale à 1,5 mg et inférieure ou égale à 18 mg, par rapport à 1 g de la membrane à fibres creuses.

**3.** Dispositif de purification de sang basé sur une membrane à fibres creuses selon la revendication 1, dans lequel le taux d'attachement de l'eau constituant le mélange d'eau et d'alcool polyhydrique à la membrane à fibres creuses est supérieur ou égal à 40 % et inférieur ou égal à 100 % par rapport au poids sec de la membrane à fibres creuses, et le taux d'attachement de l'alcool polyhydrique à la membrane à fibres creuses est supérieur ou égal à 20 % et inférieur ou égal à 300 %, par rapport au poids sec de la membrane à fibres creuses, dans lequel les rapports d'attachement de l'eau et de l'alcool polyhydrique sont déterminés comme décrit dans la description.

FIG. 1

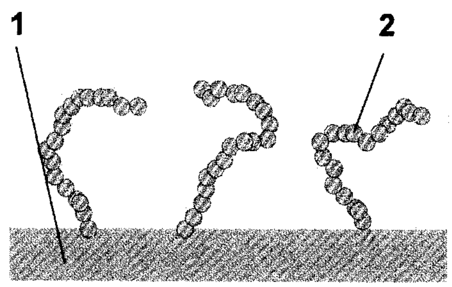

FIG. 2

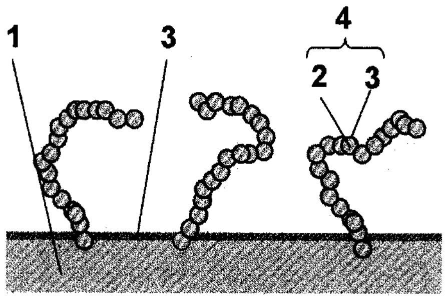

FIG. 3

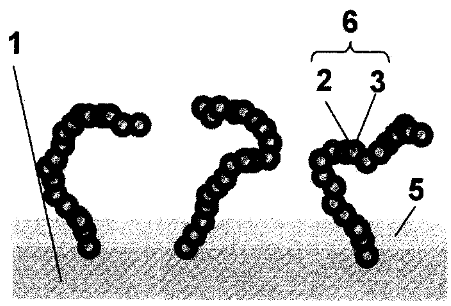

FIG. 4

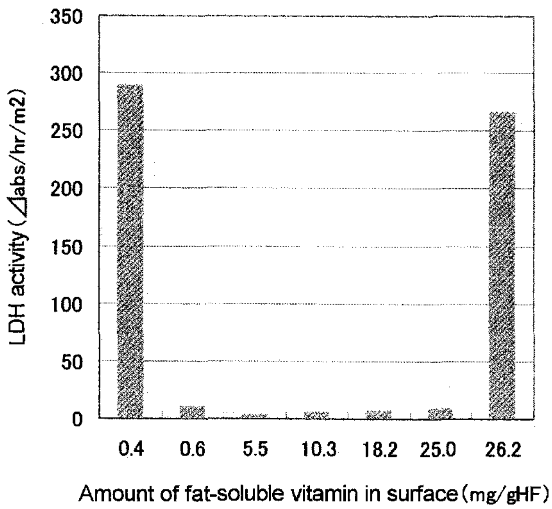
350
300
250
200
150
100
50
0
LDH activity (⊿abs/hr/m2)
0.4
0.6
5.5
10.3
18.2
25.0
26.2
Amount of fat-soluble vitamin in surface (mg/gHF)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7178166 A
- JP 2006296931 A
- JP 2008093228 A
- EP 0923955 A
- JP 4038583 B